# FASCICULE DE BREVET EUROPEEN

(11) **EP 0 650 958 B1**
(45) Date de publication et mention de la délivrance du brevet: **25.02.1998**
(21) Numéro de dépôt: 94402169.0
(22) Date de dépôt: 29.09.1994
(51) Int. Cl.: A61K 31/175, C07D 207/32, C07D 213/87, A61K 31/44

(54) **Dérivés d'indane triones et d'hydrazides et leur application thérapeutique**
Hydrazone von Indantrione und ihre therapeutische Verwendung
Hydrazones of indane-triones and their application in therapy

(30) Priorité: 01.10.1993 FR 9311720
(43) Date de publication de la demande: 03.05.1995
(73) Titulaire: LABORATOIRE INNOTHERA Société Anonyme, 94111 Arcueil (FR)
(72) Inventeur: Desquand, Stéphanie, F-75015 Paris (FR); Finet, Michel, F-94260 Fresnes (FR); Le Marquer, Florence, F-75005 Paris (FR); Tembo, Norbert Olivier, F-95800 Cergy Saint Christophe (FR); Torregrosa, Jean-Luc, F-93200 Saint Denis (FR); Yannic-Arnoult, Sylvie, F-91350 Grigny (FR)
(74) Mandataire: Dupuis-Latour, Dominique

(56) Documents cités:
- EP-A- 0 566 445
- EP-A- 0 566 446
- CHEMICAL ABSTRACTS, vol. 96, no. 17, 26 Avril 1982, Columbus, Ohio, US; abstract no. 142649z, S.D. FEDORYAK page 738 ;
- CHEMICAL ABSTRACTS, vol. 112, no. 11, 12 Mars 1990, Columbus, Ohio, US; abstract no. 98342s, R. IQBAL ET AL. page 732 ;

## Description

La présente invention concerne des dérivés d'indane-triones et d'hydrazides et leurs applications thérapeutiques.

Chemical Abstracts, Vol. 112, 990, référence 11298342s enseigne des produits de condensation d'hydrazides d'acides pyridine-carboxyliques isomères avec la ninhydrine. Il est notamment indiqué dans ce document le 2,3-dihydro-1,3-dioxo-2-(2-pyridyl) carbonylhydrazono(1H)-indène.

Les dérivés d'indane-triones et d'hydrazides selon la présente invention répondent à la formule générale :

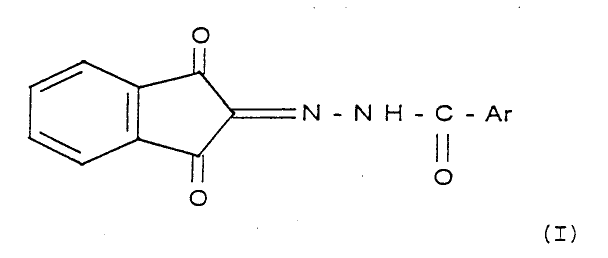

Chemical Abstracts, vol. 96, abstract nº 142649z décrit (composé(II)) un dérivé particulier, éventuellement salifié, dans lequel Ar est un radical 4-pyridyle.

Le EP-A-0 566 466, publié postérieurement à la présente demande et donc opposable au seul titre de la nouveauté, décrit des dérivés particuliers où Ar est un radical aminophényle (exemple 47) ou un radical 2-pyrazinyle (exemple 44).

La présente invention a pour objet des sels nouveaux et de nouvelles utilisations en thérapeutique.

L'activité des sels nouveaux selon la présente invention est nettement améliorée par rapport à l'état de la technique.

Selon la présente invention, dans le composé de formule générale (I) ci-dessus le radical Aᵣ est un radical pyrrol-1-yle.

La présente invention concerne également des sels des composés salifiables mentionnés plus haut. Ces sels comprennent les sels d'addition d'acides minéraux tels que l'acide chlorhydrique, bromhydrique, sulfurique, phosphorique ou nitrique et les sels d'addition d'acides organiques tels que l'acide acétique, propionique, oxalique, citrique, maléique, fumarique, succinique et tartrique.

La présente invention vise les sels d'addition d'acides minéraux et d'acides organiques selon la formule (I) dans lesquels le radical Aᵣ est un radical 3-pyridyle.

La présente invention concerne aussi l'utilisation des composés mentionnés ci-dessus pour l'obtention d'un médicament destiné au traitement de l'insuffisance veineuse fonctionnelle et organique, des pathologies hémorroïdaires, des infections inflammatoires ostéoarticulaires, dermatologiques et cardiovasculaires et au traitement des états de chocs constitués par une chute importante de la pression artérielle plus particulièrement dans les états de chocs septiques.

L'invention est illustrée par les exemples non limitatifs ci-après :

### exemple 1

### 2.3-Dihydro-1.3-dioxo-2-(pyrrol-1-yl-carbonylhydrazono)-1H-indene

A une solution de 0.570g (3.2 mmoles) de 2.3-dihydro-2.2-dihydroxy-1.3-dioxo-1H-indene dans 5ml d'éthanol, on ajoute 0.400g (3.2 mmoles) de pyrrol-1-yl-carbonylhydrazine en solution dans l'eau.
Le mélange réactionnel est agité à température ambiante pendant 10 minutes et le précipité blanc formé est filtré, puis remis en suspension dans 10 ml d'éthanol. A cette suspension on ajoute deux gouttes d'acide chlorhydrique concentré puis l'on porte le mélange à 60°C pendant deux heures.
Le précipité orange obtenu est essoré à chaud, lavé à l'éther éthylique et séché.
Cristaux jaunes
**Rdt :** 50%
**F :** 203°C
**Rf :** 0.6 (CH₂Cl₂/MeOH : 97/3)
**RMN du** ^{**1**}**H (270 MHz, DMSO d**_{**6**}**) : d (ppm)**
   13.37 (ls, 1H, NH)
   8.13 (s, 4H, H-4, H-5, H-6, H-7)
   7.52 (m, 2H, H-2', H-5' )
   6.55 (m, 2H, H-3', H-4')
**SM** (I.E) : m/z 267 (M^{+.})
**-IR (KBr)**
   ν NH : 3220 cm⁻¹, C=O : 1748 et 1690 cm^{-1.}

### exemple 2

### 2.3-Dihydro-1.3-dioxo-2-[(3-pyridyl)carbonylhydrazono]-1H-indene, chlorhydrate

A 400mg (1.4 mmoles) de 2.3-dihydro-1.3-dioxo-2-[3-(3-pyridyl)carbonylhydrazono]-1H-indene en solution dans 20 ml de dichlorométhane on ajoute à 0°C 0.07ml (1.4 mmoles) d'acide chlorhydrique et 10ml d'ether, le sel formé qui précipite est filtré sur verre fritté, rinçé à l'éther puis au pentane et séché une nuit sous vide à 50°C.
**-Rdt :** 100%
**-F :** >260°C
**-Rf :** 0.55 (CH₂Cl₂-MeOH, 97 : 3)
**-RMN du** ^{**1**}**H (270 MHz, DMSO d**_{**6**}**) : d (ppm)**
   13.38 (ls, 1H, NH)
   9.15 (s, 1H, H-2')
   8.93 (d, 1H, H-6', J5'-6'=4.9Hz)
   8.44 (d, 1H, H-4', J4'-5'=7.9Hz)
   8.04 (s, 4H, H-4, H-5, H-6, H-7)
   7.81 (m, 1H, H-5')
**-IR (KBr)**
   ν C=O : 1732, 1718 et 1681 cm⁻¹

### exemple 3

### 2.3-Dihydro-1.3-dioxo-2-[(3-pyridyl)carbonylhydrazono]-1H-indene, (L)-tartrate

A 0.6g (2.15 mmoles) de 2.3-dihydro-1.3-dioxo-2-[(3-pyridyl)carbonylhydrazono]-1H-indene en solution dans 100 ml de dichlorométhane, on ajoute à température ambiante 0.322g (2.15 mmoles) d'acide tartrique. Le mélange réactionnel précipite aprés 10 minutes pour fournir 0.922 g de 2.3-dihydro-1.3-dioxo-2-[(3-pyridyl) carbonylhydrazono]-1H-indene, tartrate sous forme de cristaux jaunes.
**-Rdt** : 100%
**-F :** 100°C
**-Rf :** 0.58 (CH₂Cl₂-MeOH, 97 : 3)
**-RMN du** ^{**1**}**H (270 MHz, DMSO d**_{**6**}**) : d (ppm)**
   11.35 (ls, 1H, NH)
   11.13 (ls, 1H, COOH)
   9.10 (ls, 1H, H-2')
   8.89 (d, 1H, H-6', JH-5'-6'=4.73Hz)
   8.30 (d, 1H, H-4', JH4'-5'=7.48Hz)
   8.05 (ls, 4H, H-4, H-5, H-6, H-7)
   7.83 (ls, 2H, 2xOH)
   7.57 (m, 1H, H-5')
   4.33 (ls, 2H, 2xCHOH)
   3.89 (ls, 1H, NH+)
**-IR (KBr)**
   ν NH : 3300 cm⁻¹, C=O : 1734 et 1687cm⁻¹

### exemple 4

### 2.3-Dihydro-1.3-dioxo-2-[(3-pyridyl)carbonylhydrazono]-1H-indene, maleate

A 0.3g (1.07 mmoles) de 2.3-dihydro-1.3-dioxo-2-[(3-pyridyl)carbonylhydrazono]-1H-indene en solution dans 100 ml de dichlorométhane, on ajoute à température ambiante 0.12g (1.07 mmoles) d'acide maleique. Le mélange réactionnel précipite pour fournir 0.422 g de 2.3-dihydro-1.3-dioxo-2-[(3-pyridyl)carbonylhydrazono]-1H-indene, maleate sous forme de cristaux jaunes.
**-Rdt :** 100%
**-F :** 206°C
**-Rf :** 0.58 (CH₂Cl₂-MeOH, 97 : 3)
**-RMN du** ^{**1**}**H (270 MHz, DMSO d**_{**6**}**) : d (ppm)**
   13.42 (ls, 1H, NH)
   11.13 (ls, 1H, COOH)
   9.08 (ls, 1H, H-2')
   8.89 (d, 1H, H-6', JH-5'-6'=3.97Hz)
   8.29 (d, 1H, H-4', JH4'-5'=7.94Hz)
   8.04 (ls, 4H, H-4, H-5, H-6, H-7)
   7.69 (m, 1H, H-5')
   6.27 (ls, 2H, CH=CH)
   3.89 (ls, 1H, NH+)
**-IR (KBr)**
   ν NH : 3300 cm⁻¹, C=O : 1732 et 1687cm⁻¹

### exemple 5

### 2.3-Dihydro-1.3-dioxo-2-[(3-pyridyl)carbonylhydrazono]-1H-indene, succinate

A 0.6g (2.15 mmoles) de 2.3-dihydro-1.3-dioxo-2-[(3-pyridyl)carbonylhydrazono]-1H-indene en solution dans 100 ml de dichlorométhane, on ajoute à température ambiante 0.253g (2.15 mmoles) d'acide succinique. Le mélange réactionnel précipite pour fournir 0.397 g de 2.3-dihydro-1.3-dioxo-2-[(3-pyridyl)carbonylhydrazono]-1H-indene, succinate sous forme de cristaux jaunes.
**-Rdt :** 100%
**-F :** 162°C
**-Rf :** 0.58 (CH₂Cl₂-MeOH, 97 : 3)
**-RMN du** ^{**1**}**H (270 MHz, DMSO d**_{**6**}**) : d (ppm)**
   13.51 (ls, 1H, NH)
   12.26 (ls, 1H, COOH)
   9.17 (ls, 1H, H-2')
   8.96 (d, 1H, H-6', JH5'-6'=4.42Hz)
   8.38 (d, 1H, H-4', JH4'-5'=7.93Hz)
   8.12 (ls, 4H, H-4, H-5, H-6, H-7)
   7.76 (m, 1H, H-5')
   3.89 (ls, 1H, NH+)
   2.59 (ls, 4H, 2xCH2)
**-IR (KBr)**
   ν NH : 3300 cm⁻¹, C=O : 1735 et 1690cm⁻¹

### exemple 6

### 2.3-Dihydro-1.3-dioxo-2-[(3-pyridyl)carbonylhydrazono]-1H-indene, sulfate

A 400mg (1.4 mmoles) de 2.3-dihydro-1.3-dioxo-2-[(3-pyridyl)carbonylhydrazono]-1H-indene en solution dans 20 ml de dichlorométhane, on ajoute à 0°C 0.07ml (1.4 mmoles) d'acide sulfurique, le sel formé qui précipite est filtré sur verre fritté, rinçé à l'ether puis au pentane et séché une nuit sous vide à 50°C.
**-Rdt :** 100%
**-F :** 240°C
**-Rf :** 0.52 (CH₂Cl₂-MeOH, 97 : 3)
**-RMN du** ^{**1**}**H (270 MHz, DMSO d**_{**6**}**) : d (ppm)**
   13.39 (ls, 1H, NH)
   9.09 (s, 1H, H-2')
   8.88 (d, 1H, H-6', J6-5=4.9Hz)
   8.45 (d, 1H, H-4', J5-6=7.9Hz)
   8.03 (s ,4H, H-4, H-5, H-6, H-7)
   7.82 (m, 1H, H-5')
   7.55 (ls, 2H, NH+)
**-IR (KBr)**
   ν C=O : 1732, 1718 et 1681 cm⁻¹

Les composés selon la présente invention et leurs sels ont montré qu'ils possèdent diverses propriétés pharmacologiques. Ainsi, ils sont veinotoniques et n'affectent pas dans la plupart des cas le système artériel ; par ailleurs, ils augmentent la résistance capillaire, diminuent l'hyperperméabilité vasculaire induite par certains agents inflammatoires et présentent des propriétés antilipoperoxydantes, antiradicalaires et antiinflammatoires, ainsi qu'une activité protectrice dans le choc septique.

Ces propriétés sont démontrées chez les mammifères tels que les rats, cobayes et lapins, dans des conditions in vitro (vaisseaux ou réseaux vasculaires isolés) et in vivo.

Pour l'étude in vitro, les composés sont solubilisés en solution aqueuse pure ou contenant du DMSO ou de l'alcool.

Pour l'étude in vivo, ils sont administrés par voie intra-veineuse sous forme de solution aqueuse pure, par voie intrapéritonéale sous forme de solution aqueuse contenant ou non du DMSO ou par voie orale en solution ou en suspension dans la carboxyméthylcellulose ou dans une solution aqueuse composée contenant du Tween^{(R)} et dans certains cas du DMSO (diméthylsulfoxyde).

### Modèles d'études pharmacologiques

L'effet contractile est mesuré in vitro :
- par la force de contraction développée par des anneaux vasculaires quiescents ou stimulés (soit électriquement soit par des agents physiologiques) et maintenus dans des conditions isométriques,
- par la pression développée par des réseaux vasculaires perfusés à débit constant.

In vivo, les pressions artérielles et veineuses sont mesurées dans des conditions normales et après arrêt cardiaque. Lors de l'arrêt cardiaque, le tonus veineux est calculé à partir des pressions veineuses et artérielles mesurées à l'équilibre et corrigées en fonction des différences relatives de compliance entre ces deux réseaux (Samar et Coleman, Am. J. Physiol., 1978, 234 : H94-100 ; Yamamoto et col., Am. J; Physiol., 1980, 238 : H823-828).

L'augmentation de la résistance capillaire est appréciée par la modification de l'index pétéchial (pression négative induisant l'extravasation d'érythrocytes), mesuré par une méthode dérivée de l'angiosterromètre de Parrot.

La perméabilité vasculaire est étudiée in vivo et in vitro par la mesure de l'extravasation d'albumine ou de colorant liant l'albumine (Bleu Evans). In vivo, l'hyperperméabilité est induite par l'injection d'une solution d'histamine, de bradykinine ou de zymosan. Les modèles in vitro permettent de réaliser des hyperpressions (sur un territoire vasculaire isolé) et/ou des réactions vasculaires inflammatoires.

L'activité antiinflammatoire est démontrée par la mesure de l'inhibition de l'oedème et de la migration leucocytaire après induction d'une pleurésie chez le rat par injection de carragénine dans la cavité pleurale (Almeida et col., J. Pharmacol. Exp. Therap., 1980, 214 : 74).

L'effet "piégeur de radicaux libres" global est étudié in vitro par un modèle utilisant le 1,1-diphényl-2-picrylhydrazyl (DPPH) comme radical libre stable, méthode dérivée de celle décrite par Lamaison et col., Plantes Médic. et Phytothérapie, XXII, 1988, 231-234.

L'activité antioxydante est étudiée in vitro par un modèle de peroxydation lipidique basée sur la peroxydation d'une émulsion d'acide linoléique par le fer, méthode modifiée par rapport à celle décrite par Sutherland et col., Arch. Biochem. Biophys., 1982, 214, 1-11.

L'activité dans le choc septique est étudiée chez le rat après induction par une endotoxine lipopolysaccharidique (15 mg/kg), méthode voisine de celle décrite par Terashita et col., Eur. J. Pharmacol., 109, 257-261, 1985.

### Exemples d'effets pharmacologiques

Les composés de l'invention et leurs sels éventuels augmentent la contraction des veines saphènes animales produite par la noradrénaline et la dépolarisation (solution hyperpotassique) sans affecter, dans la majorité des cas, les réponses contractiles artérielles. A titre illustratif, les composés des exemples 2, 5 et 6 (10 nM à 30 mM) augmentent de plus de 40 % (DE₅₀ ± 0,3 microM) les contractions des veines saphènes de lapin produites par le KCl (40 mM).

Le composé de l'exemple 5 augmente à leur concentration maximale de 30 à 200 % la contraction des veines saphènes de lapin en réponse à la noradrénaline 0,3 micromolaire.

A titre illustratif, les composés des exemples 1 et 3 augmentent la résistance capillaire de base de 10 à 100 %, lorsque celle-ci est mesurée une heure à deux heures après administration de 1-20 mg/kg/i.p. et jusqu'à quatre à six heures après administration orale de 1-20 mg/kg chez le rat.

A titre illustratif, les composés des exemples 1, 4 et 6 inhibent l'hyperperméabilité vasculaire induite par le zymosan après administration orale de 1-20 mg/kg.

A titre illustratif, les composés des exemples 4 et 5 présentent un effet antiradicalaire dans le modèle utilisant le DPPH.

### Toxicité

Par ailleurs, les composés de l'invention et leurs sels éventuels sont très peu toxiques. Par exemple, après administration unique per os chez la souris, aucune mortalité n'est observée à la dose de 1 g/kg pour la majorité des composés. Les seuls effets observés sont dans certains cas des diarrhées colorées et des urines colorées, ces dernières étant le témoin d'une résorption du produit.

Ce qui précède montre que les composés de l'invention et leurs sels éventuels peuvent être utilisés en thérapeutique humaine et animale. Ils sont en particulier indiqués dans l'insuffisance veineuse fonctionnelle, organique et les pathologies hémorroïdaires par leurs composantes vasculaires et antiinflammatoires, ainsi que dans les affections typiquement inflammatoires (ostéoarticulaires, dermatologiques ou cardiovasculaires) et dans les états de chocs constitués par une chute importante de la pression artérielle, en particulier dans les états de chocs septiques (endotoxiques).

L'insuffisance veineuse fonctionnelle se caractérise par une dilatation et une hyperdistensibilité des veines superficielles des membres inférieurs, oedèmes, paresthésies à type d'impatince, de jambe sans repos. Ce type de pathologie peut évoluer vers l'insuffisance veineuse organique (varices, incontinence valvulaire profonde ...) voire vers la phlébothrombose et les lésions ulcéreuses.

Dans cette pathologie veineuse, une composante inflammatoire s'installe dans les premiers stades et se manifeste plus clairement dans les stades avancés.

La présente invention comprend donc l'utilisation des composés mentionnés ci-dessus et de leurs sels éventuels, comme substances actives pour la préparation de médicaments et compositions pharmaceutiques, à usage humain et vétérinaire, comprenant au moins un desdits composés et sels en association avec un support ou diluant physiologiquement acceptable.

La forme de ces médicaments et compositions pharmaceutiques dépendra bien évidemment de la voie d'administration souhaitée notamment orale, parentérale et rectale et ils peuvent être formulés selon les techniques classiques avec mise en oeuvre des supports et véhicules usuels.

Ainsi, dans le cas d'une administration par voie orale, ils peuvent se présenter sous la forme de comprimés, tablettes, gélules, solutions, sirops et suspensions.

Les comprimés, tablettes et gélules contiennent la substance active conjointement avec un diluant (par exemple lactose, dextrose, sucrose, mannitol, sorbitol ou cellulose), un lubrifiant (par exemple silice, talc ou stéarate comme le stéarate de magnésium), un liant (par exemple amidon, méthylcellulose ou gomme arabique), un agent de désintégration (alginate par exemple) et ils sont fabriqués par des techniques connues par exemple de mélange, de granulation, de pastillage, d'enrobage, etc ...

Les sirops peuvent contenir, à titre de support, glycérol, mannitol et/ou sorbitol. Les solutions et suspensions peuvent comprendre de l'eau et un support tel qu'une gomme naturelle, de la gélose, de l'alginate de sodium ou de l'alcool polyvinylique.

Pour l'administration par voie parentérale, les médicaments et compositions peuvent prendre la forme de solutions, d'émulsions ou de suspensions comprenant la substance active et un support approprié tel que l'eau stérile ou des solutions salines isotoniques aqueuses stériles.

Pour l'administration par voie rectale, ils peuvent prendre la forme de suppositoires comprenant la substance active et un support approprié tel que le beurre de cacao ou du polyéthylène glycol.

La dose thérapeutique des substances actives pourra atteindre 2000 mg/jour suivant la voie d'administration, l'âge, le poids et l'état du sujet à traiter et la puissance thérapeutique de la substance active mise en oeuvre.

## Revendications

1. Dérivé d'indane-triones et d'hydrazides caractérisé en ce qu'ils répond à la formule générale : 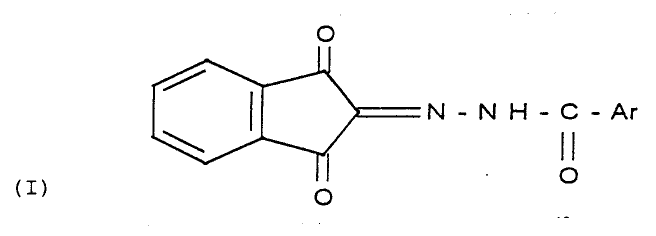 dans laquelle Ar est un radical pyrrol-1-yle.

2. Les sels d'addition d'acides minéraux choisis parmi l'acide chlorhydrique, l'acide bromhydrique, l'acide sulfurique, l'acide phosphorique et l'acide nitrique et les sels d'addition d'acides organiques choisis parmi l'acide acétique, l'acide propionique, l'acide oxalique, l'acide citrique, l'acide maléique, l'acide fumarique, l'acide succinique et l'acide tartrique des dérivés de formule générale : 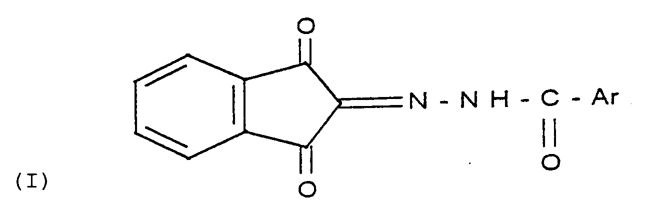 dans laquelle Ar est un radical 3-pyridyle.

3. Utilisation des dérivés selon l'une des revendications 1 et 2 pour l'obtention d'un médicament destiné au traitement de l'insuffisance veineuse fonctionnelle et organique.

4. Utilisation des dérivés selon l'une des revendications 1 et 2 pour l'obtention d'un médicament destiné au traitement des pathologies hémorroïdaires.

5. Utilisation des dérivés selon l'une des revendications 1 et 2 pour l'obtention d'un médicament destiné au traitement des infections inflammatoires ostéoarticulaires, dermatologiques et cardio-vasculaires.

6. Utilisation des dérivés selon l'une des revendications 1 et 2 pour l'obtention d'un médicament destiné au traitement des états de choc constitués par une chute importante de la pression artérielle, plus particulièrement dans les états de choc septique.

7. Utilisation du 2,3-dihydro-1,3-dioxo-2-(3-pyridyl carbonylhydrazono)1H-indène pour l'obtention d'un médicament destiné au traitement de l'insuffisance veineuse fonctionnelle et organique.

8. Utilisation du 2,3-dihydro-1,3-dioxo-2-(3-pyridyl carbonylhydrazono)1H-indène pour l'obtention d'un médicament destiné au traitement des pathologies hémorroïdaires.

9. Utilisation du 2,3-dihydro-1,3-dioxo-2-(3-pyridyl carbonylhydrazono)1H-indène pour l'obtention d'un médicament destiné au traitement des infections inflammatoires ostéoarticulaires, dermatologiques et cardiovasculaires.

10. Utilisation du 2,3-dihydro-1,3-dioxo-2-(3-pyridyl carbonylhydrazono)1H-indène pour l'obtention d'un médicament destiné au traitement des états de choc constitués par une chute importante de la pression artérielle plus particulièrement dans des états de choc septique.

## Claims

1. Hydrazones of indane-triones characterized by the general formula : 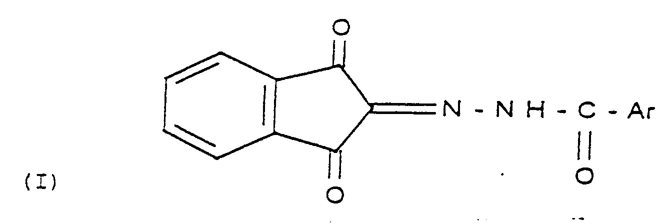 wherein Ar is a 1-pyrrolyl group.

2. Addition mineral salts selected from hydrochloric acid, bromhydric acid, sulfuric acid, phosphoric acid and nitric acid and addition organic salts selected from acetic acid, propionic acid, oxalic acid, citric acid, maleic acid, fumaric acid, succinic acid and tartric acid of compounds having the general formula : 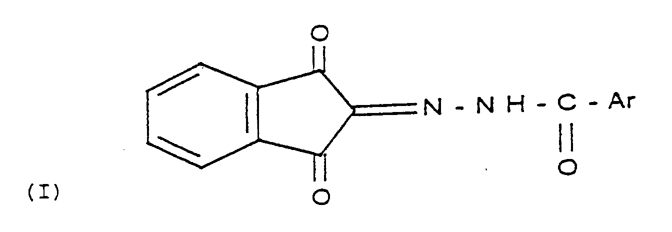 wherein Ar is a 3-pyridyl group.

3. Use of the compounds according to one of claims 1 and 2 for the manufacture of a drug for the treatment of organic and functional blood insufficiency.

4. Use of the compounds according to one of claims 1 and 2 for the manufacture of a drug for the treatment of hemorrhoidal pathologies.

5. Use of the compounds according to one of claims 1 and 2 for the manufacture of a drug for the treatment of osteoarticular, dermatological and cardio-vascular inflammatory infections.

6. Use of the compounds according to one of claims 1 and 2 for the manufacture of a drug for the treatment of shock states resulting from a significative drop of blood pressure particularly of septic shock states.

7. Use of 2,3-dihydro-1,3-dioxo-2-(3-pyridylcarbonyl-hydrazono)-1H-indene for the manufacture of a drug for the treatment of organic and functional blood insufficiency.

8. Use of 2,3-dihydro-1,3-dioxo-2-(3-pyridylcarbonyl-hydrazono)-1H-indene for the manufacture of a drug for the treatment of hemorrhoidal pathologies.

9. Use of 2,3-dihydro-1,3-dioxo-2-(3-pyridylcarbonyl-hydrazono)-1H-indene for the manufacture of a drug for the treatment of osteoarticular, dermatological and cardio-vascular inflammatory infections.

10. Use of 2,3-dihydro-1,3-dioxo-2-(3-pyridylcarbonyl-hydrazono)-1H-indene for the manufacture of a drug for the treatment of shock states resulting from a significative drop of blood pressure particularly of septic shock states.

## Patentansprüche

1. Derivate von Indantrionen und Hydraziden, dadurch gekennzeichnet, daß sie der folgenden allgemeinen Formel entsprechen: 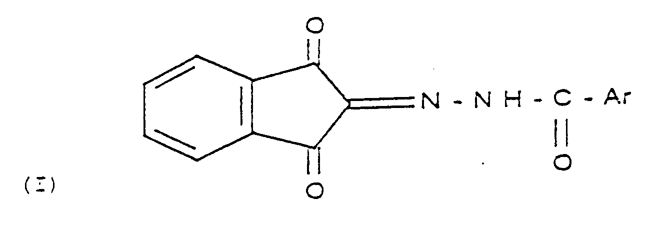 in der Ar ein Pyrrol-1-yl-Rest ist.

2. Additionssalze von Mineralsäuren ausgewählt aus Salzsäure, Bromwasserstoffsäure, Schwefelsäure, Phosphorsäure und Salpetersäure und Additionsalze von organischen Säuren ausgewählt aus Essigsäure, Propionsäure, Oxalsäure, Zitronensäure, Maleinsäure, Fumarsäure, Bernsteinsäure und Weinsäure, von Derivaten der allgemeinen Formel: 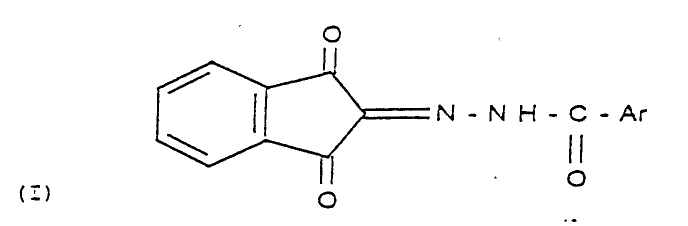 in der Ar ein 3-Pyridyl-Rest ist.

3. Verwendung der Derivate nach einem der Ansprüche 1 oder 2 zur Herstellung eines Medikaments zur Behandlung einer funktionellen oder organischen Veneninsuffizienz.

4. Verwendung der Derivate nach einem der Ansprüche 1 oder 2 zur Herstellung eines Medikaments zur Behandlung von hämorrhoidalen Krankheiten.

5. Verwendung der Derivate nach einem der Ansprüche 1 oder 2 zur Herstellung eines Medikaments zur Behandlung von entzündlichen osteoartikulären, dermatologischen und kardiovaskulären Infektionen.

6. Verwendung der Derivate nach einem der Ansprüche 1 oder 2 zur Herstellung eines Medikaments zur Behandlung von Schockzuständen, die durch einen bedeutenden arteriellen Druckabfall gebildet werden, insbesondere bei der Behandlung von septischen Schockzuständen.

7. Verwendung von 2,3-Dihydro-1,3-dioxo-2-(3-pyridylcarbonylhydrazon)1H-inden zur Herstellung eines Medikaments zur Behandlung einer funktionellen oder organischen Veneninsuffizienz.

8. Verwendung von 2,3-Dihydro-1,3-dioxo-2-(3-pyridylcarbonylhydrazon)1H-inden zur Herstellung eines Medikaments zur Behandlung von hämarrhoidalen Krankheiten.

9. Verwendung von 2,3-Dihydro-1,3-dioxo-2-(3-pyridylcarbonylhydrazon)1H-inden zur Herstellung eines Medikaments zur Behandlung von entzündlichen osteoartikulären, dermatologischen und kardiovaskulären Infektionen.

10. Verwendung von 2,3-Dihydro-1,3-dioxo-2-(3-pyridylcarbonylhydrazon)1H-inden zur Herstellung eines Medikaments zur Behandlung von Schockzuständen, die durch einen bedeutenden arteriellen Druckabfall gebildet werden, insbesondere bei der Behandlung von septischen Schockzuständen.
